(19) Europäisches Patentamt
European Patent Office
Office européen des brevets

(11) Numéro de publication : **0 177 742 B1**

(12) # FASCICULE DE BREVET EUROPÉEN

(45) Date de publication du fascicule du brevet : 07.03.90

(21) Numéro de dépôt : 85111014.8

(22) Date de dépôt : 31.08.85

(51) Int. Cl.[5] : C 07 C211/25, C 07 D295/02, C 07 C 49/637, C 07 C 69/145, C 11 B 9/00, A 61 K 7/46

(54) **Composés azotés, leur préparation et leur utilisation à titre de produits de dèpart pour la préparation de cétones décaliniques.**

(30) Priorité : 12.09.84 CH 4354/84

(43) Date de publication de la demande : 16.04.86 Bulletin 86/16

(45) Mention de la délivrance du brevet : 07.03.90 Bulletin 90/10

(84) Etats contractants désignés : CH DE FR GB LI

(56) Documents cités :
GB--A-- 1 209 398
US--A-- 3 932 515
US--A-- 3 988 373
THE JOURNAL OF ORGANIC CHEMISTRY, vol. 47, no. 3, 1982, pages 389-397, Columbus, Ohio, US; K.A. PARKER et al.: "Reactions of propargyl alcohols with amide acetals"
THE JOURNAL OF ORGANIC CHEMISTRY, vol. 45, no. 2, 1980, pages 216-236, Columbus, Ohio, US; O.D. DAILEY JR. et al.: "Synthesis of a model for the BCE ring system of bruceantin. A caveat on the cyclohexene -- trans diaxial diol conversion"
JOURNAL OF THE AMERICAN CHEMICAL SOCIETY, vol. 102, no. 12, 4 juin 1980, pages 4274-4275, formule 3; J.A. MARSHALL et al.: "Total synthesis of (+,-)-Dihydrospiniferin-1:A furanosesquiterpene with a 1,6-methano[10]annulene carbon skeleton"

(73) Titulaire : FIRMENICH SA
1, route des Jeunes
CH-1211 Genève 8 (CH)

(72) Inventeur : Snowden, Roger Leslie, Dr.
5, chemin des Palettes
CH-1212 Grand-Lancy (CH)

(74) Mandataire : Salvadori, Giuseppe, Dr.
c/o Firmenich S.A. Case Postale 239
CH-1211 Genève 8 (CH)

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 177 742 B1

Jouve, 18, rue St-Denis, 75001 Paris, France

## Description

La présente invention a trait à certains composés azotés utiles en tant que produits de départ pour la préparation de cétones décaliniques, ces derniers composés étant des intermédiaires avantageux dans la préparation d'ingrédients parfumants prisés.

Les composés azotés, dont il est fait mention ici, appartiennent à la classe de composés de formule

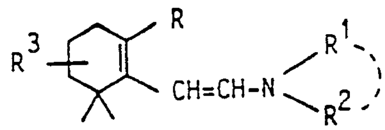

(I)

dans laquelle le symbole R désigne un groupe alkyle $C_1$-$C_3$, $R^1$ et $R^2$ représentent, chacun pris séparément, un radical alkyle $C_1$-$C_6$ ou, pris conjointement, un polyméthylène et $R^3$ représente un groupe alkyle $C_1$-$C_3$, de préférence méthyle, ou l'hydrogène.

Les symboles R et $R^3$ servent donc à définir un radical méthyle, éthyle, n-propyle, iso-propyle. De préférence R représente un méthyle ou un éthyle, tandis que $R^3$ représente de préférence un méthyle.

Les substituants $R^1$ et $R^2$ peuvent représenter, pris séparément, méthyle, éthyle, n-propyle, iso-propyle, n-butyle, sec-butyle, tert-butyle, pentyle ou hexyle, ou, pris conjointement, un polyméthylène. De préférence, ils servent à définir un radical méthyle ou un tétraméthylène ; dans ce dernier cas, ils forment, avec l'atome d'azote auquel ils sont liés, un groupe pyrrolidinyle.

Enfin le substituant $R^3$ peut être lié au noyau cyclohexénique à l'une quelconque des positions permises. C'est ainsi que la formule (I) sert à définir, par exemple, l'un des composés préférentiels suivants :

[2-(2,6,6-triméthyl-1-cyclohexène-1-yl)-1-éthényl]-pyrrolidine,
[2-(2-éthyl-6,6-diméthyl-1-cyclohexène-1-yl)-1-éthényl]-pyrrolidine,
N,N-diméthyl-[2-(2,6,6-triméthyl-1-cyclohexène-1-yl)-1-éthényl]-amine,
N,N-diméthyl-[2-(2-éthyl-6,6-diméthyl-1-cyclohexène-1-yl)-1-éthényl]-amine,
[2-(2,3,6,6-tétraméthyl-1-cyclohexène-1-yl)-1-éthényl]-pyrrolidine,
[2,(2-éthyl-3,6,6-triméthyl-1-cyclohexène-1-yl)-1-éthényl]-pyrrolidine,
[2-(2-éthyl-4,6,6-triméthyl-1-cyclohexène-1-yl)-1-éthényl]-pyrrolidine,
[2-(2-éthyl-5,6,6-triméthyl-1-cyclohexène-1-yl)-1-éthényl]-pyrrolidine,
[2-(2,4,6,6-tétraméthyl-1-cyclohexène-1-yl)-1-éthényl]-pyrrolidine,
[2-(2,5,6,6-tétraméthyl-1-cyclohexène-1-yl)-1-éthényl]-pyrrolidine,
N,N-diméthyl-[2-(2,3,6,6-tétraméthyl-1-cyclohexène-1-yl)-1-éthényl]-amine,
N,N-diméthyl-[2-(2,4,6,6-tétraméthyl-1-cyclohexène-1-yl)-1-éthényl]-amine,
N,N-diméthyl-[2-(2,5,6,6-tétraméthyl-1-cyclohexène-1-yl)-1-éthényl]-amine,
N,N-diméthyl-[2(2-éthyl-3,6,6-triméthyl-1-cyclohexène-1-yl)-1-éthényl]-amine,
N,N-diméthyl-[2-(2-éthyl-4,6,6-triméthyl-1-cyclohexène-1-yl)-1-éthényl]-amine,
N,N-diméthyl-[2-(2-éthyl-5,6,6-triméthyl-1-cyclohexène-1-yl)-1-éthényl]-amine.

L'invention concerne également un procédé pour la préparation des composés de formule (I), caractérisé en ce qu'on additionne une amine de formule

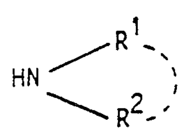

(II)

à un aldéhyde de formule

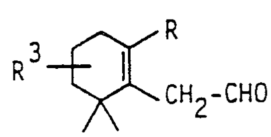

(III)

dans lesquelles les symboles R, $R^1$, $R^2$ et $R^3$ ont le sens indiqué pour la formule (I).

Les composés de formule (I) sont des entités chimiques nouvelles. Ils constituent une série de composés particulièrement utiles en tant que produits de départ pour la préparation de cétones décaliniques de formule

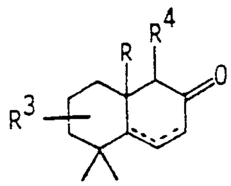

(IV)

contenant une double liaison dans l'une des positions indiquées par les pointillés et dans laquelle $R^4$ définit un atome d'hydrogène ou un méthyle, et les symboles R et $R^3$ ont le sens indiqué pour la formule (I), lesquelles cétones servent à la préparation d'esters décaliniques odorants employés dans l'industrie de la parfumerie (voir le brevet suisse n° 536804).

Les composés azotés (I) sont transformés en cétones (IV) suivpant un procédé original caractérisé en ce que :

a. on additionne lesdits composés azotés à une oléfine de formule

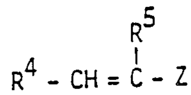

(V)

dans laquelle chacun des symboles $R^4$ et $R^5$ représente un atome d'hydrogène ou un radical méthyle et Z définit un radical —CN ou —C(O)$OR^6$, dans lequel $R^6$ sert à désigner un radical alkyle, de préférence $C_1$-$C_4$, ou aralyphatique, de préférence benzyle ;

b. on traite le composé résultant à l'aide d'anhydride acétique ou on le soumet à un traitement thermique pour fournir le composé de formule

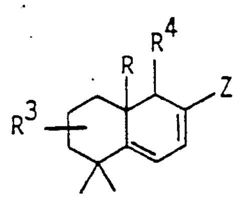

(VI)

c. on traite ledit composé au moyen d'un hydroxyde alcalin, de préférence en solution alcoolique, pour donner un acide de formule

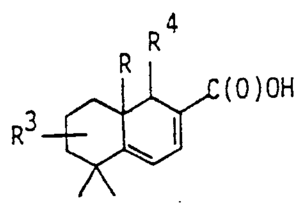

(VII)

d. on transforme enfin l'acide (VII) résultant en la cétone (IV) désirée par une réaction de dégradation de type usuel.

Cette dernière étape, qui en soi procède de manière analogue à celle des méthodes décrites dans l'art, peut consister à traiter l'acide (VII) au moyen de chlorure de thionyle, par exemple, à faire réagir le chlorure d'acyle correspondant qui en résulte avec de l'azoture de sodium, à soumettre l'azoture formé à l'action de la chaleur et à traiter enfin à chaud en milieu acide l'isocyanate formé. La méthode suivie dite de type Curtius est décrite dans le détail dans les exemples qui suivent. D'autres méthodes connues dans l'art peuvent également être employées avec succès à une telle transformation. L'étape b. peut s'effectuer par deux voies distinctes. L'une qui consiste à traiter le produit résultant de l'étape a. à l'aide d'anhydride acétique ; l'autre qui consiste à soumettre ledit produit à une action thermique, par exemple à une température supérieure à 150 °C, comprise de préférence entre 150 et 200 °C.

Les cétones (IV) sont des composés nouveaux. Il s'agit de composés utiles en tant que produits de départ pour la préparation d'esters décaliniques de formule

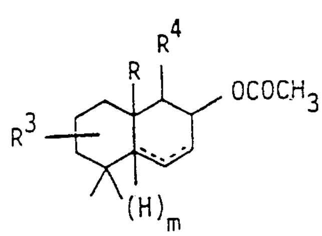

(VIIIa)

pouvant contenir une double liaison dans l'une des positions indiquées par les pointillés et dans laquelle les substituants R, $R^3$ et $R^4$ ont le sens indiqué plus haut, et l'indice m vaut zéro ou un. Les esters contenant une double liaison sont obtenus par réduction de la fonction cétonique à l'aide d'un réactif usuel, tel le borhydrure de sodium ou l'aluminohydrure de diisobutyle, suivie de l'estérification du carbinol obtenu.

Lorsqu'on effectue par contre une réduction desdites cétones par hydrogénation catalytique, par exemple sous pression et en présence de nickel de Raney, on obtient un mélange isomérique des esters décaliniques saturés.

Dans les deux cas, lesdits esters (VIIIa) peuvent se présenter sous différentes formes isomériques. Leur forme prédominante cependant est la forme décalinique trans (env. 80 %) définie à l'aide de la formule que voici :

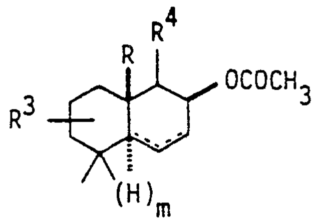

(VIIIc)

Ces esters sont des composés nouveaux, à l'exeption de l'acétate de perhydro-5,5,8a-triméthyl-2-naphtyle qui est décrit dans GB-P-1 209 398, et possèdent des propriétés odorantes fort avantageuses. Ils sont de ce fait très prisés en tant qu'ingrédients parfumants pour la préparation de parfums et produits parfumés. Ils développent des notes olfactives boisées de grande finesse et trouvent un emploi dans une variété de compositions parfumantes de natures diverses.

L'invention est illustrée à l'aide des exemples suivants dans lesquels les températures sont indiquées en degrés centigrades et les abréviations ont le sens usuel dans l'art. Les spectres RMN ont été effectués sur des échantillons des produits à l'examen en solution dans le deutérochloroforme.

Exemple 1

1,2,4a,5,6,7,8,8a-octahydro-5,5,7a cis-triméthyl-naphtalène-2-one,
1,2,3,5,6,7,8,8a-octahydro-5,5,8a-triméthyl-naphtalène-2-one et
1,2,4a,5,6,7,8,8a-octahydro-5,5,8a trans-triméthyl-naphtalène-2-one

Un mélange de 11 g (0,05M) d'acide 5,5,8a-triméthyl-1,5,6,7,8,8a-hexahydronaphtalène-carboxylique et 8,9 g (0,075 M) de $SOCl_2$ a été soumis à reflux pendant 3 h. Après évaporation de l'excès de $SOCl_2$ sous pression réduite ($2 \times 10^3$ Pa), on a distillé le résidu à 26,6 Pa pour obtenir le chlorure d'acyle désiré sous forme d'une huile jaune pâle (11,3 g ; Eb. 120-122°/26,6 Pa).

Une solution du chlorure d'acyle dans 30 ml d'acétone a été ajoutée goutte à goutte pendant 20 min à une solution agitée d'azoture de sodium (4,3 g ; 66 mM) dans 20 ml d'eau à 0-5°. La température du mélange a été ensuite portée à 25° en l'espace d'1 h et maintenue à cette même valeur pendant 2 h supplémentaires. L'extraction avec 3 fractions de 30 ml chacune de toluène et le traitement usuel ont permis d'obtenir l'azoture d'acyle en solution de toluène (40-50 ml). Après séchage azéotropique, on ajoute goutte à goutte la solution, pendant 20 min, à 60 ml de toluène maintenus sous agitation à 100-110° pour former l'isocyanate correspondant.

A la solution ainsi obtenue, maintenue à 100°, on ajoute goutte à goutte, et pendant 5 min, 5 ml d'HCl aqueux.

La réaction, qui s'accompagne d'un dégagement rapide de $CO_2$, se termine en 75-80 min environ. Le mélange de réaction a été ensuite neutralisé avec une solution aqueuse saturée de $NaHCO_3$, extrait, soumis aux traitements habituels et distillé (Eb. 82-92°/26,6 Pa). La fraction ainsi obtenue était constituée par un mélange de trois cétones désirées sous forme d'une huile incolore (7,1 g ; rend. 74 %).

L'isolation des trois produits purs a été effectuée par chromatographie sur colonne (support : $SiO_2$ ; éluant : cyclohexane/acétate d'éthyle).

1,2,3,5,6,7,8,8a-octahydro-5,5,8a-triméthyl-naphtalène-2-one
IR : 1725, 1460, 1380, 1320, 1265, 1230, 990, 850, 650 $cm^{-1}$ ;
RMN : 1;10 (6H) ; 1,16 (3H) ; 1,0—2,0 (6H) : 2,30 (ABqa, J = 13Hz, 2H) ; 2,87 (m, 2H) ; 5,57 (t, J = 4Hz, 2H) delta ppm ;
SM : $M^+$ = 192(37) ; m/e : 177(17), 150(30), 135(100), 122(14), 107(29), 93(31), 79(25).

1,2,4a,5,6,7,8,8a-octahydro-5,5,8a cis-triméthyl-naphtalène-2-one
RMN ($^1$H) : 0,88 ; 1,00 ; 1,07 (3s, 9H) ; 1,2-1,6 (6H) ; 2,3 (ABqa ; J = 18Hz, 2H) ; 6,11 (d, J = 10Hz, 1H) ; 6,98 (dxd, J = 10 et 6Hz, 1H) delta ppm ;

RMN ($^{13}C$) : 200,6s ; 150,6d ; 129,6d ; 53,0d ; 46,4t ; 41,3t ; 40,2t ; 35,0s ; 32,8qa ; 31,7qa ; 23,1qa ; 18,8t delta ppm ;
SM : $M^+$ = 192(8) ; m/e : 177(4), 149(19), 121(13), 109(100), 91(9), 79(18).

1,2,4a,5,6,7,8,8a-octahydro-5,5,8-a trans-triméthyl-naphtalène-2-one
IR : 1680, 1460, 1380, 1250, 1170, 880, 820, 720 $cm^{-1}$ ;
RMN ($^1H$) : 0,91, 1,00 et 1,03 (3s, 9H) ; 1,2-1,8 (6H), 2,21 (m, 2H) ; 6,07 (dxd, J = 10 et 3Hz, 1H) ; 6,98 (dxd, J = 10 et 2Hz, 1H) delta ppm ;
RMN ($^{13}C$) : 199,7s ; 150,5d ; 130,2d ; 58,0t ; 54,6d ; 41,5t ; 40,4t ; 39,8s ; 32,5qa ; 22,0qa ; 18,8qa, 18,5t delta ppm ;
SM : $M^+$ = 192(25) ; m/e : 177(7), 149(14), 135(15), 122(70), 109(100), 81(45).

L'acide de départ a été préparé ainsi :

a. (2,6,6-triméthyl-1-cyclohexène-1-yl)-acétaldéhyde
Une solution de 100 g de 1-éthinyl-2,6,6-triméthyl-1-cyclohexanol (0,6 M) (voir brevet suisse n° 636009) dans 500 ml de xylène et 5 g de silylvanadate, obtenu comme indiqué ci-après, a été maintenue à reflux pendant 23 h. Après évaporation du solvant, on a obtenu par distillation fractionnée du résidu l'aldéhyde désiré sous forme d'une huile incolore (77 g ; rend. 77 %) ; Eb. 109-110°/2 × $10^3$ Pa.
Silylvanadate. Une solution de dichlorure de diphénylsilyle (40,5 g ; 0,16 M) dans l'acétone (130 ml) a été ajoutée goutte à goutte à 25° pendant 20 min. à une solution d'orthovanadate de sodium ($Na_3VO_4$) (10,1 g ; 0,05 M) dans 150 ml d'eau. La température du mélange réactionnel augmentait ainsi jusqu'à 35-40° pendant que l'agitation était maintenue pendant 10 min. Puis le tout a été refroidi dans un bain à glace. 350 ml d'eau glacée ont été ajoutés et le mélange a été agité pendant 5 min., puis filtré. Le solide recueilli a été lavé deux fois à l'eau et séché pà 66,5 Pa sur du $CaCl_2$ anhydre. Rend. 35,1 g.

b. N,N-diméthyl-[2-(2,6,6-triméthyl-1-cyclohexène-1-yl)-1-éthényl]-amine
Un mélange de 5 g (0,03 M), 25 ml de toluène et 3,7 g (0,033 M) d'une solution aqueuse à 40 % de diméthylamine a été mélangé à 60° pendant 1 h. La phase organique a été séparée, lavée avec de l'eau et avec une solution aqueuse concentrée de NaCl et séchée sur $Na_2SO_4$ anhydre. Après évaporation du solvant, une distillation fractionnée du résidu a permis d'obtenir 5,63 g (rend. 97 %) de l'amine désirée ayant Eb. 120° temp. du bain)/1,33 Pa.
(IR 1640, 1450, 1350, 1140, 1080, 940, 780 et 718 $cm^{-1}$ ; RMN : 0,99 (6H) ; 1,3-2,2 (9H) ; 2,61 (s, 6H) ; 4,58 (large d, J = 14 Hz, 1H) ; 5,82 (d, J = 14 Hz, 1H) delta ppm ; SM : $M^+$ = 193(100) ; m/e : 178(71), 133(17), 122(29), 108(26).
La réaction procède de manière analogue en utilisant le xylène à la place du toluène.

c. 5,5,8a-triméthyl-1,5,6,7,8,8a-hexahydro-naphtalène-carboxylate de méthyle
Une solution de 3,86 g (20 mM) de l'amine obtenue suivant b. ci-dessus, 3,44 g (40 mM) d'acrylate de méthyle dans 20 ml de toluène contenant 20 mg d'hydroquinone a été chauffée dans une autoclave en acier inox sous azote à 150° pendant 3 jours. Puis 4,1 g (40 mM) d'anhydride acétique ont été ajoutés et le mélange a été chauffé à nouveau à 150° pendant 1 h. L'évaporation du solvant et une distillation ont permis d'obtenir 3,95 g (rend. 84 %) de l'ester désiré, Eb. 160-180°/2,66 Pa.
IR : 1710, 1570, 1460, 1440, 1260, 1225, 1100, 850, 740 et 670 $cm^{-1}$ ;
RMN : 1,03 (s, 3H) ; 1,16 (6H) ; 1,2-2,0 (6H) ; 2,31 (2H, ABqa, J = 16Hz) ; 3,76 (s, 3H), 6,0 (d, J = 6Hz, 1H) ; 7,0 (dxd, J = 6 et 2,5Hz, 1H) delta ppm ;
SM : $M^+$ = 234(50) ; m/e : 219(31), 164(100), 149(67), 119(50), 105(86), 91(54).

d. acide 5,5,8a-triméthyl-1,5,6,7,8,8a-hexahydro-naphtalène-carboxylique
Une solution de 2,34 g (10 mM) de l'ester obtenu suivant c. ci-dessus, dans 5 ml de méthanol, a été ajoutée goutte à goutte à une solution agitée de KOH (1,7 g ; 30 mM) dans 15 ml de méthanol à 25°. Le mélange a été mis à reflux pendant 5 h puis, après évaporation du méthanol, on a ajouté de l'eau. Après acidification avec HCl aqueux, extraction à l'acétate d'éthyle et traitement usuel des extraits organiques, on a obtenu 2,06 g (rend. 94 %) de l'acide désiré sous forme d'un solide cristallin, F. 184-185,5°.
IR ($CDCl_3$) : 3050, 1670, 1630, 1560, 1420, 1280, 1230, 854, 820 $cm^{-1}$ ;
RMN : 1,03 (s, 3H) ; 1,16 (6H) ; 1,2-2,0 (6H) ; 2,20 (ABqa, J = 16Hz, 2H) ; 6,05 (d, J = 6Hz, 1H) ; 7,16 (dxd, J = 6 et 2,5 Hz, 1H) ; 11,25 (large s, 1H) delta ppm ;
SM : $M^+$ = 220(35) ; m/e : 205(18), 150(100), 135(60), 119(30), 105(75), 91(88).
Le même cycle d'opérations peut être effectué en remplaçant l'acrylate de méthyle par l'acrylate de n-butyle et l'acrylate de benzyle sans changement notable des résultats observés.

Exemple 2

1,2,3,5,6,7,8,8a-octahydro-5,5,6,8a-tétraméthyl-naphtalène-2-one
1,2,4a,5,6,7,8,8a-octahydro-5,5,6,8a-tétraméthyl-naphtalène-2-one

5 g (0,021 M) d'acide 1,5,6,7,8,8a-hexahydro-5,5,6,8a-tétraméthyl-naphtalène-carboxylique ont été soumis à une dégradation de Type Curtius conformément à l'Exemple 1 pour fournir 3,4 g (rend. 77 %) du mélange de cétones désiré. Une chromatographie sur colonne (éluant : cyclohexane/acétate d'éthyle) sur un support de silice a permis d'obtenir 0,63 g d'une huile incolore constituée par 1,2,3,5,6,7,8,8a-octahydro-5,5,6,8a beta-tétraméthyl-naphtalène-2-one ayant Eb. 150°(bain)/6,6 Pa.

IR : 1730, 1680, 1460, 1380, 1240, 1010, 840 et 678 $cm^{-1}$ ;

RMN : 0,79 (d, J = 7Hz, 3H) ; 0,97 (s, 3H) ; 1,10 et 1,20 (2s, 6H), 1,00-2,50 (5H), 2,30 (ABq, J = 13Hz, 2H) ; 2,87 (t, J = 4Hz, 2H) ; 5,57 (t, J = 4Hz, 1H) delta ppm ;

SM : $M^+$ = 206 ; m/e : 191 (23), 164 (24), 149 (100), 122 (47), 107 (57).

L'acide de départ a été préparé ainsi :

a. (2,5,6,6-tétraméthyl-1-cyclohexényl)-acétaldéhyde

Une solution de 2,2,3,6-tétraméthyl-1-éthynyl-1-cyclohexanol (15 g ; 0,083 M) dans 100 ml de xylène a été maintenue à reflux pendant 20 h en présence de 2 g de vanadate de diphenylsilyle. Après évaporation du xylène et distillation du résidu on a obtenu 13 g (rend. 87 %) de l'aldéhyde désiré.

Eb. 48-52°/6,65 Pa.

IR : 2730, 1725, 1460, 1390, 1380, 1364, 1070 et 1040 $cm^{-1}$.

b. 1,5,6,7,8,8a-hexahydro-5,5,6,8a-tétraméthyl-naphtalène-carboxylate de méthyle

Un mélange de 13 g (0,072 M) de l'aldéhyde obtenu sous lettre a., 15,4 ml (0,12 M) de diméthylamine à 40 % et 70 ml de xylène a été mélangé à 90° pendant 3 h. La séparation des phases a été suivie d'extraction au xylène de la phase aqueuse et des traitements usuels de lavage et séchage. Après filtration, à la solution claire obtenue, on a ajouté 13,5 g (0,16 M) d'acrylate de méthyle et le mélange a été chauffé à 180° pendant 24 h dans un autoclave inox. L'évaporation du solvant et une distillation du résidu ont permis d'obtenir 13,2 g (rend. 74 %) d'une huile incolore ayant Eb. 112-115°/4 Pa.

IR : 1710, 1566, 1440, 1380, 1096, 854, 742 et 680 $cm^{-1}$.

c. acide 1,5,6,7,8,8a-hexahydro-5,5,6,8a-tétraméthyl-naphtalène-carboxylique

Une solution de 12,5 g (0,05 M) de l'ester obtenu sous lettre b. ci-dessus, et de 6,5 g de KOH (0,12 M) dans 100 ml de méthanol a été chauffée à reflux pendant 4 h. Après évaporation du méthanol, de l'eau a été ajoutée au résidu, puis la solution alcaline a été extraite à l'éther et la phase aqueuse acidifiée avec de l'HCl aqueux à 10 %.

Le produit brut désiré a été recueilli par filtration et lavage avec de l'eau et de l'acétone suivi de séchage sur du $P_2O_5$ sous vide. Rend. 94 % ; 11 g. F. 151-182°

IR (KBr) : 2900, 1670, 1625, 1555, 1420, 1275, 1090, 1040, 845, 745 et 670 $cm^{-1}$ ;

RMN : 0,85-0,90 (2t, J = 7Hz ; 3H) ; 0,92 ; 0,95 ; 1,06, 1,13 et 1,18 (9H) ; 1,25-1,70(4H), 2,05 (1H) ; 2,18 (2ABq, J = 16Hz, 2H) ; 5,98 et 6,08 (2d, J = 6Hz ; 1H) ; 6,90 (1H) delta ppm ;

SM : $M^+$ = 234 ; m/e : 220 (19), 163 (29), 150 (100), 135 (46), 119 (39), 105 (82), 91 (79).

Exemple 3

1,2,3,5,6,7,8,8a-octahydro-5,5-diméthyl-8a-éthyl-naphtalène-2-one

Cette naphtalénone a été préparée suivant la méthode décrite à l'Exemple 2 à partir de 2,2-diméthyl-6-éthyl-1-éthynyl-1-cyclohexanol.

## Revendications

1. Composés azotés de formule

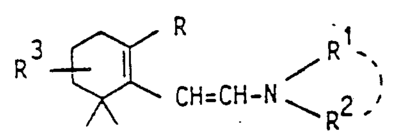

(I)

dans laquelle le symbole R désigne un groupe alkyle $C_1$-$C_3$, $R^1$ et $R^2$ représentent, chacun pris séparément, un radical alkyle $C_1$-$C_6$ ou, pris conjointement, un polyméthylène et $R^3$ représente un groupe alkyle $C_1$-$C_3$, de préférence méthyle, ou l'hydrogène.

2. Composés selon la revendication 1, dont la formule contient un substituant R qui désigne un groupe méthyle, et $R^1$ et $R^2$, pris conjointement, désignent un radical $-(CH_2)_4$ ou chacun, pris séparément, désigne un radical méthyle.

3. Composés selon la revendication 1, dont la formule contient un substituant R qui désigne un groupe éthyle, et $R^1$ et $R^2$, pris conjointement, désignent un radical $-(CH_2)_4$ ou chacun, pris séparément, désigne un radical méthyle.

4. Procédé pour la préparation des composés selon la revendication 1, caractérisé en ce qu'on additionne une amine de formule

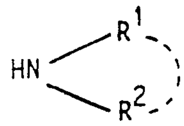

(II)

à un aldéhyde de formule

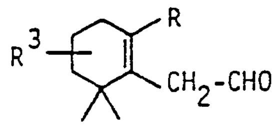

(III)

dans lesquelles les symboles R, $R^1$, $R^2$ et $R^3$ ont le sens indiqué pour la formule (I).

5. Utilisation des composés azotés selon la revendication 1 à titre de produits de départ pour la préparation de cétones décaliniques de formule

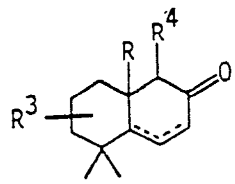

(IV)

contenant une double liaison dans l'une des positions indiquées par les pointillés et dans laquelle $R^4$ définit un atome d'hydrogène ou un méthyle, et les symboles R et $R^3$ ont le sens indiqué pour la formule (I), caractérisée en ce que :

a. on additionne lesdits composés azotés à une oléfine de formule

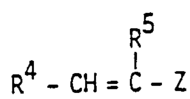

(V)

dans laquelle chacun des symboles $R^4$ et $R^5$ représente un atome d'hydrogène ou un radical méthyle et Z définit un radical —CN ou —C(O)OR$^6$, dans lequel $R^6$ sert à désigner un radical alkyle, de préférence $C_1$-$C_4$, ou aralyphatique ;

b. on traite le composé résultant à l'aide d'anhydride acétique ou on le soumet à un traitement thermique pour fournir le composé de formule

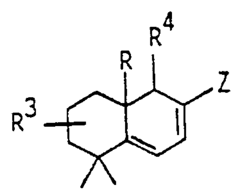

(VI)

c. on traite ledit composé au moyen d'un hydroxyde alcalin pour donner un acide de formule

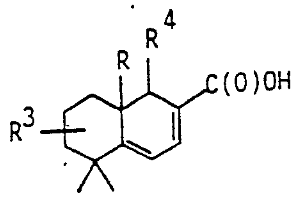

(VII)

d. on transforme enfin l'acide (VII) résultant en la cétone (IV) désirée par une réaction de dégradation de type usuel.

6. Composés cétoniques de formule

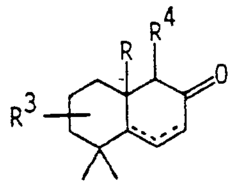

(IV)

contenant une double liaison dans l'une des positions indiquées par les pointillés et dans laquelle R désigne un groupe alkyle $C_1$-$C_3$, $R^3$ représente un groupe alkyle $C_1$-$C_3$, de préférence méthyle, ou l'hydrogène, et $R^4$ définit un atome d'hydrogène ou un méthyle.

**Claims**

1. Nitrogen containing compounds of formula

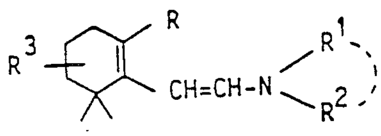

(I)

wherein symbol R designates a $C_1$-$C_3$ alkyl group, each of $R^1$ and $R^2$ represents, when taken separately, a $C_1$-$C_6$ alkyl radical, or when taken together, a polymethylene and $R^3$ represents a $C_1$-$C_3$ alkyl group, preferably a methyl radical, or a hydrogen atom.

2. Compounds according to claim 1 wherein substituent R of formula (I) designates a methyl radical, and $R^1$ and $R^2$, taken together, designate a $-(CH_2)_4$ radical or each of them, taken separately, stands for a methyl radical.

3. Compounds according to claim 1 wherein substituents R of formula (I) designates an ethyl radical, and $R^1$ and $R^2$, taken together, designate a $-(CH_2)_4$ radical or each of them, taken separately, stands for a methyl radical.

4. Process for the preparation of the compounds according to claim 1 characterized in that it comprises the addition of an amine of formula

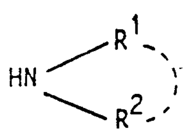

(II)

to an aldehyde of formula

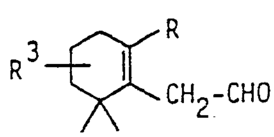

(III)

wherein symbols R, $R^1$, $R^2$ and $R^3$ have the meaning given for formula (I).

5. Utilization of the nitrogen containing compounds according to claim 1 as starting products for the preparation of decalin ketones of formula (I)

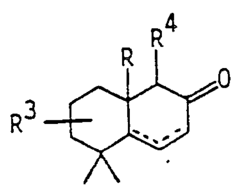

(IV)

containing a double bond in one of the positions indicated by the dotted lines and wherein $R^4$ designates a hydrogen atom or a methyl radical and symbols R and $R^3$ have the meaning given for formula (I), characterized in that :

a. the said nitrogen containing compounds are added to an olefin of formula

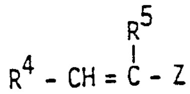

(V)

wherein each of symbols $R^4$ and $R^5$ represents a hydrogen atom or a methyl radical and Z designates a —CN radical of a —C(O)OR$^6$, wherein $R^6$ stands for an alkyl radical, preferably a $C_1$-$C_4$ alkyl, or an araliphatic radical ;

b. the resulting compound is treated with acetic anhydride or, is subjected to a thermal treatment to give a compound of formula

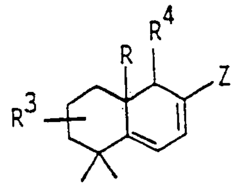

(VI)

c. the said compound is treated with an alkali metal hydroxide to give an acid of formula

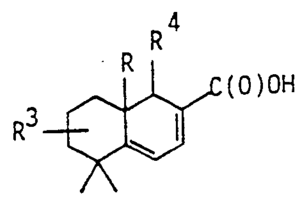

(VII)

d. finally, the acid (VII) is converted into the desired ketone (IV) by a conventional degradation reaction.

6. Ketone compounds of formula

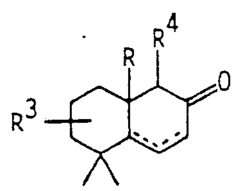

(IV)

containing a double bond in one of the positions indicated by the dotted lines and wherein R designates a $C_1$-$C_3$ alkyl group, $R^3$ represents a $C_1$-$C_3$ alkyl, preferably a methyl radical, or a hydrogen atom, and $R^4$ designates a hydrogen atom or a methyl radical.

**Patentansprüche**

1. Stickstoffhaltige Verbindungen der Formel,

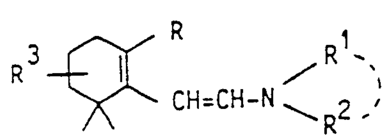

(I)

in welcher das Symbol R ein $C_1$ bis $C_3$ Alkylradikal bedeutet, jedes der Symbole $R^1$ und $R^2$ getrennt genommen ein $C_1$ bis $C_6$ Alkylradikal, oder zusammen genommen ein Polymethylen darstellen, und $R^3$ ein $C_1$ bis $C_3$ Alkylradikal, vorzugsweise ein Methylradikal oder ein Wasserstoffatom bedeutet.

2. Verbindungen gemäß Anspruch 1, worin die Formel einen Substituenten R enthält, welcher ein Methylradikal bedeutet, und die Symbole $R^1$ und $R^2$ zusammen genommen ein $-(CH_2)_4-$ Radikal darstellen, oder jedes der $R^1$ und $R^2$ getrennt genommen ein Methylradikal bedeutet.

3. Verbindungen gemäß Anspruch 1, worin die Formel einen Substituenten R enthält, welcher ein

Aethylradikal bedeutet, und die Symbole $R^1$ und $R^2$ zusammen genommen ein $-(CH_2)_4$ Radikal darstellen, oder jedes der $R^1$ und $R^2$ getrennt genommen ein Methylradikal bedeutet.

4. Verfahren zur Herstellung der Verbindungen gemäß Anspruch 1, dadurch gekennzeichnet, daß man ein Amin der Formel

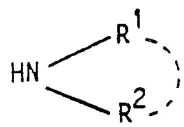

(II)

an einen Aldehyd der Formel,

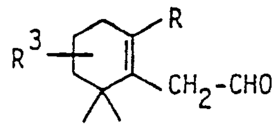

(III)

in welcher die Symbole R, $R^1$, $R^2$ und $R^3$ den für die Formel (I) angegebenen haben, addiert.

5. Verwendung der stickstoffhaltigenen Verbindungen gemäß Anspruch 1 als Ausgangsprodukte zur Herstellung von Dekalin-Ketonen der Formel,

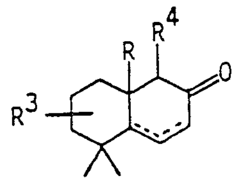

(IV)

die in einer der durch die gestrichelten Linien angegebenen Stellungen eine Doppelbindung hat, und worin $R^4$ ein Wasserstoffatom oder ein Methylradikal bedeutet, und die Symbole R und $R^4$ den für Formel (I) angegebenen haben, dadurch gekennzeichnet, daß man :

a. die obengenannten stickstoffhaltigen Verbindungen an ein Olefin der Formel,

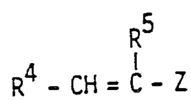

(V)

in welcher jedes der Symbole $R^4$ und $R^5$ ein Wasserstoffatom oder ein Methylradikal darstellt, und Z ein —CN oder —C(O)$OR^6$ Radikal bedeutet, worin $R^6$ vorzugsweise für ein $C_1$ bis $C_4$ Alkylradikal oder für ein araliphatisches Radikal steht, addiert ;

b. die so erhaltene Verbindung mit Essigsäureanhydrid behandelt oder sie einer thermischen Behandlung unterwirft, um die Verbindung der Formel

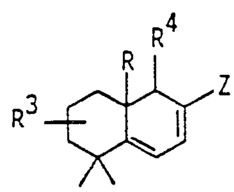

(VI)

zu erhalten ;

c. die entsprechende Verbindung mit einem alkalischen Hydroxyd behandelt, um eine Säure der Formel

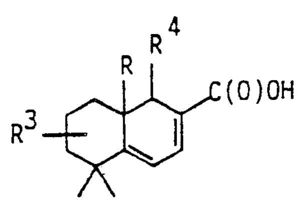

(VII)

zu erhalten ;

d. endlich, die resultierende Säure (VII) mittels einer typischen Abbaureaktion in das gewünschte Keton verwandelt.

6. Ketonische Verbindungen der Formel,

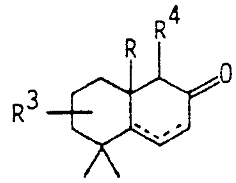

(IV)

die in einer der durch die gestrichelten Linien angegebenen Stellungen eine Doppelbindung enthält und in welcher R ein $C_1$ bis $C_3$ Alkylradikal bedeutet, $R^3$ für ein $C_1$ bis $C_3$ Alkylradikal, vorzugsweise ein Methylradikal oder ein Wasserstoffatom steht, und $R^4$ ein Wasserstoffatom oder ein Methylradikal darstellt.